# EUROPEAN PATENT SPECIFICATION

(11) **EP 0 267 258 B1**
(45) Date of publication and mention of the grant of the patent: **07.04.1993**
(21) Application number: 87903390.0
(22) Date of filing: 13.05.1987
(51) Int. Cl.: A61B 5/14, A61M 5/32

(54) **NEEDLE FOR MULTIPLE VACUUM BLOOD SAMPLE DEVICES**
KANÜLE FÜR VORRICHTUNGEN ZUM ANSAUGEN VON MEHRFACHEN BLUTPROBEN DURCH UNTERDRUCK
AIGUILLE POUR DISPOSITIFS DE PRELEVEMENT SOUS-VIDE D'ECHANTILLONS SANGUINS MULTIPLES

(30) Priority: 14.05.1986 IT 488386; 30.12.1986 IT 362786
(43) Date of publication of application: 18.05.1988
(73) Proprietor: Celli, Goffredo, I-47040 Cerasolo Ausa-Coriano (FO) (IT)
(72) Inventor: Roberti, Lamberto, I-61100 Pesaro (IT)
(74) Representative: Lanzoni, Luciano
(86) International application number: PCT/IT87/00044
(87) International publication number: WO 87/06813

(56) References cited:
- US-A- 3 434 468
- US-A- 3 520 292
- US-A- 4 409 990

## Description

The present invention concerns a needle for multiple vacuum blood sample devices, pointed at both opposite ends to form two needle portions, the first of which is designed to be introduced into the epidermis and vein, and the second to be introduced into the closing stopper of a test tube; the two needle portions are coplanar but not coaxial, and are connected by an intermediate lenght oblique to the needle portions.

As is well known in medical technique, blood samples for tests are taken using common syringes fitted with a hollow needle for sucking the blood out of the vein.

Simple as these operations might seem when carried out by an expert, it is known that it is occasionally not so easy to introduce the needle in the vein without passing right through the vein itself, this making it necessary for the operation to be carried out again from the very beginning.

The above happens as a result of the fact that the syringe always takes up a certain amount of space, obliging the operator to carry out the injection and above all introduction into the vein with a certain angle of incidence with respect to the surface of the epidermis.

Syringes are also in existance that have a decentred or eccentric needle attachment point: in these cases it is easier for the blood to be taken, but it can only be transferred to the test tube after the syringe has been withdrawn from the vein. Often, however, more than one test is carried out, and it is thus necessary for several samples to be taken and divided out into as any test tubes. This thus forces one to use syringes with notable dimensions in order to take the necessary quantity of blood in one operation for subsequently filling the single test tubes.

The problem of the angle of incidence of the needle, and that of taking multiple blood samples, are both solved by the use of a device composed of a test tube carrying cylinder (the said "holder") designed to receive vacuum test tubes, of a first needle, held at one end by a small pipe featuring two flexible diameterical tangs enabling it to be gripped by hand, and of a second needle, which can be connected to the other end of the small pipe by means of what is known as a "LUER" cone, and its point inserted in a static mode within the test tube carrying cyclinder. This second needle is fitted with a rubber cap that covers it. The test tubes are closed under vacuum with rubber stoppers that are less thick in their centre portion in order to facilitate their being pierced by the second needle. Using this device it is possible to introduce the first needle into the vein and practically parallel to it, in that the space taken up by its diameter is almost nil. It is then sufficient to place a test tube in the test tube carrying cylinder, pressing its stopper against the second needle which pierces it in its central, less thick, zone.

The full test tube can subsequently be replaced with another one without any problems, since the blood is retained by the cap on the second needle, which extends once again, completely covering the second needle once the filled test tube has been removed.

A device constructed in this way reveals itself to be very practical in terms of ease of use, but has the disadvantage that there is a notable waste of valuable material, such as the two needles, the "LUER" cone and small pipe, for every blood sample taken.

The greatest disadvantage, however, consists in the fact that the blood has to follow a long route from the vein to the test tube, with the possibility of very small clots being formed, that influence the results of the tests, above all in the case of coagulation-related tests such as the Quick time.

To eliminate these disadvantages, and above all that of the possible formation of very small blod clots, consideration has been given to the use of double needles, that is to say featuring two points, for introduction into the vein and introduction into the test tube stoppers respectively, fitted with a threaded element for connection to the test tube carrying cylinder, and featuring horizontally placed holding and opposing grip tangs for the index and middle fingers of the hand.

With this device, however, the problem of effecting a certain angle of inclination of the the needle with repect to the epidermis returns.

US-A-3434468 discloses a cannula which is used with a holder. This cannula comprises two substantially parallel needle portions, which are connected by diagonal intermediate portion.

The needle-epidermis inclination problem also brings a functional problem in its wake, represented by the fact that, when in the vein, the point of the needle ends up being too close to the opposite wall of the vein. This means that, when the blood is being sucked out of the vein, this wall is also subjected to a certain amount of suction, such that it moves closer to and comes into contact with the point of the needle itself, the blood sample taking operation thus being interrupted.

In this case, the provision of a decentred or eccentric connection for the test tube carrying cylinder cannot be contemplated, since the inner needle point (with cap) would not be able to pierce the centre portion of the test tube stopper, and piercing would be made difficult by the stopper wall being thicker.

In addition to this, there is a further considerable problem: the blood flows from the vein into the test tube at rather a high speed when using the vacuum test tube technique for taking blood samples, as a result of the difference in pressure between the two.

This speed is even higher at the start of filling the test tube since, the vacuum being at its greatest, there is maximum suction. It is thus possible on certain specific occasions, depending on the vacuum level in the test tubes, and the length of the latter, for the outflowing blood to strike against the bottom of the test tube, creating the conditions for a phenomenon commonly known as blood stress to arise.

Amongst the morphological components of the blood one finds the erythrocytes which are, as is known, composed of round-shaped cells covered by a thin membrane, and having a red-orange colour as a result of the hemoglobin contained within them.

When they are subjected to a mechanical action provoking the breakage of the membrane, the hemoglobin flows out and, mixing with the plasma, changes the transparency characteristics of the latter, making the results of certain common clinical tests unreliable.

The aim of the present invention, as described in the claims, is to eliminate all the aforementioned disadvantages, avoiding the possibility of the above-described disadvantage occurring above under all circumstances, and yet at the same time making the operation for taking multiple blood samples from a patient both easy to carry out, convenient and practical.

This aim is achieved by the present invention as a result of the special shape of the double type needle for multiple vacuum blood sample devices, which features two needles that are coplanar but not coaxial, such that the axes of the test tube carrying cylinder and that of the needle to be introduced into the vein are not coincident.

The two needles advantageously lie in the vertical plane, perpendicular to the test tube carrying cylinder's grip tangs.

A further result achieved by the present invention is that the needle within the test tube is inclined with respect to the geometrical axis of the latter, in such a way as to optimise the conditions of impact of the blood with the wall whilst the blood sample is being taken.

The invention is described in further detail hereinafter, with the help of drawings that illustrate it purely by way of example, in a form that is in no way binding.

Figure 1 is an exploded side view of the present needle in its individual components.

Figure 2 is a side view with some parts in section in order to show others, of the present needle connected to a multiple vacuum blood sample device that is ready to take a sample.

It can be clearly seen from reference to the above-mentioned drawings that the needle, shown complete by number 16, is of the double needle type, that is to say with a point at either end, forming needles 1 and 2 respectively. Needle 1, the first of these two needles 1 and 2, is designed to be introduced into the epidermis and, subsequently, the vein, and the second, 2, to be introduced into a test tube carrying cylinder 9.

An element 5 is fitted to the needle 16 in a more or less central zone, joining and retaining an element 6 for covering the first needle 1, and a threaded element 7 featuring a ring-shaped body 10, preferably having the form of a truncated cone.

The threaded element 7 has two thread starts that are offset by 180° and are designed to enage in the corresponding thread of a hole 8 that is centred with respect to the cylinder 9. The truncated cone ring-shaped body 10 has a double function: to act as a stop against the outside edges of the hole 8, and to join to and retain a second element 11 covering needle 2.

The test tube carrying cylinder 9 is designed to contain test tubes 4 that are closed under vacuum with respective stoppers 3, the wall of which is less thick in its central part and may easily be pierced by the second needle 2.

A rubber cap 17 is pulled on over the second needle 2 making a tight fit on the threaded element 7.

Needles 1 and 2 conform with present invention, being coplanar but not coaxial.

Needles 1 and 2 are connected to one another by means of a length 12 that is oblique to and connected to them. Needle 16 is bent in such a way that in the horizontal plane it lies perpendicular to the horizontal development of the grip tangs 13 on the test tube carrying cylinder 9, when the the needle is fitted to the cylinder 9 itself.

To the further advantage of the design, the retaining element 5 and threaded element 7 on the two sides of the oblique length 12, are connected to one another by means of an intermediate length 15 which covers and protects the oblique length 12 itself.

A hold-tight element 14, engaging with the covering element 6, is located between retaining element 5 and intermediary length 15.

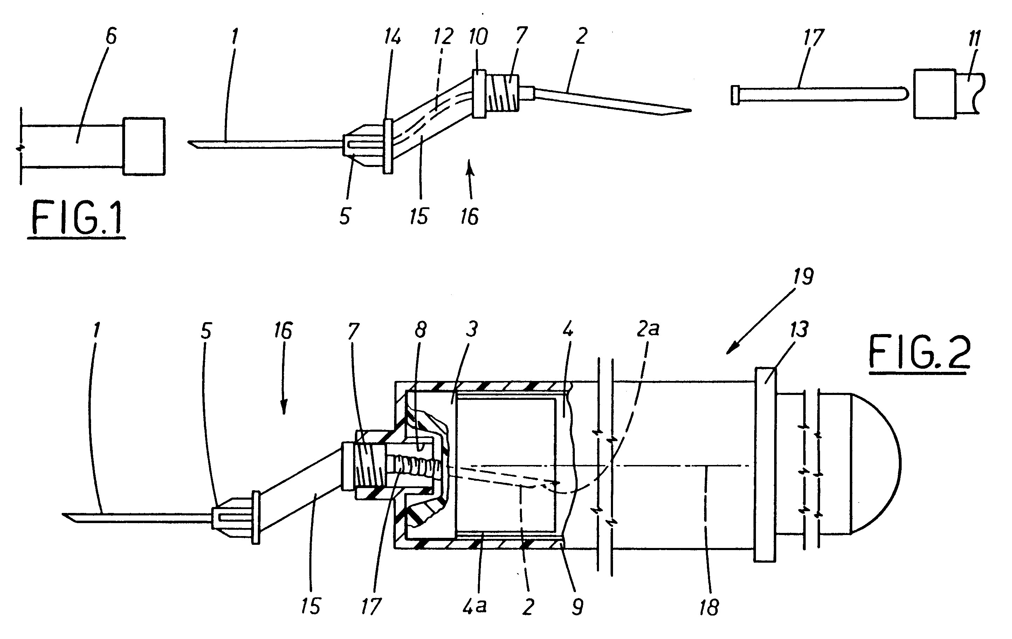

As shown in fig. 2, the cap 17 is designed to be pushed up against the threaded element 7 when a test tube 4 is introduced into the test tube carrying cylinder 9. It effects its main action, however, when there is no test tube 4 in the test tube carrying cylinder 9, by preventing blood from flowing out of the second needle 2.

It is now clear that the rigid length 15, by being oblique, performs the role of moving needle 1 down away from the axis of the test tube carrying cylinder 9, enabling the blood sample device, shown complete by number 19, to be positioned with respect to the vertical plane passing between the axes of needles 1 and 2, to obtain the optimum angle of incidence to the vein.

Although needle 2 is coplanar with the geometrical axis 18 of the test tube carrying cylinder or test tube, it is advantageously inclined downwards with respect to the latter.

Needle 2, in addition, features a cut 2a, also running downwards, along a plane at a right angle to the vertical plane passing through the axes of needles 1 and 2.

The inclination of needle 2 makes it possible for the outflowing blood to strike only the side wall 4a of the test tube.

It is obvious that by making the inclination of needle 2 compatible with the length of the test tube, so that its inclination ensures the flow of blood strikes only the side wall 4a with the minimum angle of incidence, the inevitable impact against the test tube 4, can be mitigated to such a degree that blood stress is prevented.

This effect is further reduced by the fact that needle 2 faces downwards, that is to say in the direction that decreases the number of possible impacts against the walls, whilst the orientation of cut 2a contributes towards slowing down the flow, decreasing the energy of impact against the wall.

In this way the invention, as described above and claimed hereinafter, satisfies the aims proposed.

## Claims

1. A needle for multiple vacuum blood sample devices pointed at both opposite ends to form two needle portions (1, 2); wherein the first needle portion (1) is designed to be introduced into the epidermis and vein, and the second to be introduced into the closing stopper (3) of a test tube (4); the two needle portions (1, 2) are coplanar but not coaxial, and are connected by an intermediate length (12) oblique to the needle portions characterised in that both needle portions are fitted with relative covering elements (6 and 11); the needle comprises in its substantially central portion, an element (5) for joining and retaining one of said covering elements (6), and a threaded body (7), designed to engage in a corresponding hole (8) centred in a test tube carrying cylinder (9) with the axis (18) parallel to the first needle portion (1); the said second needle (2) being inclined with respect to said axis (18) of the said test tube (4) so that in use the outflowing blood strikes only the side wall (4a) of the test tube.

2. A needle as in claim 1, **wherein** the said intermediary length (12) is located within a protective element (15) placed between the said joining and retaining element (5), featuring a ring-shaped edge (14) for holding the said covering element (6) tight, and a ring-shaped body (10) for joining and retaining the relevant said covering element (11), acting as a stop and holding the threaded body (7) tightly engaged with the said test tube carrying cylinder (9)

3. A needle as in claim 3, **wherein** the said ring-shaped body (10) has the form of a truncated cone.

4. A needle as in claim 1, **wherein** the said second needle (2) is inclined downwards towards the axis of needle portion (1) and features a cut (2a) facing downwards and running along a plane at right angles to the plane passing through the axes of the said needles (1) and (2).

## Patentansprüche

1. Kanüle für Vorrichtungen zum Ansaugen von mehrfachen Blutproben durch Unterdruck, zugespitzt an beiden sich gegenüberliegenden Enden, um zwei Kanülenabschnitte (1, 2) zu bilden, bei welcher der erste Kanülenabschnitt (1) dazu bestimmt ist, in die Haut und die Vene eingeführt zu werden, und der zweite Kanülenabschnitt (2) in den Verschlußstopfen (3) eines Reagensglases (4); wobei die beiden Kanülenabschnitte (1, 2) koplanar, jedoch nicht koaxial angeordnet und durch ein Zwischenstück (12) miteinander verbunden sind, das gegenüber den Kanülenabschnitten geneigt ist; **dadurch gekennzeichnet,** dass beide Kanülenabschnitte mit entsprechenden Abdeckelementen (6 und 11) versehen sind; und dass die Kanüle in ihrem im wesentlichen mittleren Abschnitt ein Element (5) zum Verbinden und Halten eines der genannten Abdeckelemente (6) enthält sowie einen Gewindekörper (7), der dazu bestimmt ist, in eine entsprechende Bohrung (8) eingeschraubt zu werden, die in einen das Reagensglas haltenden Zylinder (9) zentriert eingearbeitet ist, und zwar mit seiner Achse parallel zu dem ersten Kanülenabschnitt (1), wobei die genannte zweite Kanüle (2) im Verhältnis zu der genannten Achse (18) des genannten Reagensglases (4) geneigt ist, so dass während der Benutzung das ausfliessende Blut nur die Seitenwand (4a) des Reagensglases berührt.

2. Kanüle nach Patentanspruch 1, **dadurch gekennzeichnet,** dass das genannte Zwischenstück (12) in einer Schutzhülle (15) gelagert ist, angeordnet zwischen dem genannten Verbindungs- und Halteelement (5), das einen ringförmigen Rand (14) zum dichtschliessenden Halten des genannten Abdeckelementes (6) aufweist, und einem ringförmigen Körper (10) zum Verbinden und Halten des genannten entsprechenden Abdeckelementes (11), welches als Anschlag wirkt und den Gewindekörper (7) dicht abschliessend trägt, der in den genannten, das Reagensglas haltenden Zylinder (9) eingeschraubt ist.

3. Kanüle nach Patentanspruch 2, **dadurch gekennzeichnet,** dass der genannte ringförmige Körper (10) stumpfkegelförmig ausgebildet ist.

4. Kanüle nach Patentanspruch 1, **dadurch gekennzeichnet,** dass die genannte zweite Kanüle (2) nach unten in Richtung der Achse des ersten Kanülenabschnittes (1) geneigt ist und eine nach unten zeigende und entlang einer Ebene verlaufende Schneide (2a) aufweist, die im rechten Winkel zu der Ebene ausgerichtet ist, die durch die Achsen der genannten Kanülen (1) und (2) geht.

## Revendications

1. Aiguille pour dispositifs de prélévement sous-vide d'échantillons sanguins multiples, du type pointu aux deux extrémités opposées de manière à former deux portions d'aiguille (1, 2); où la première portion d'aiguille (1) est destinée à étre introduite dans l'épiderme et dans la veine et la seconde à étre introduite dans le bouchon de fermeture (3) d'une éprouvette (4); les deux portions d'aiguille (1, 2) sont coplanaires mais ne sont pas coaxiales, et sont reliées par un tronçon intermédiaire (12) oblique par rapport aux deux portions d'aiguille, caractérisée en ce que les portions d'aiguille sont toutes les deux pourvues de respectifs éléments de couverture (6 et 11); l'aiguille comporte dans sa portion sensiblement centrale un élément (5) pour l'emboîtement et la retenue de l'un (6) desdits éléments de couverture, et un corps fileté (7) destiné à s'engager dans un trou correspondant (8) prévu centré dans un cylindre de support d'une éprouvette (9) dont l'axe (18) est parallèle à la première portion d'aiguille (1); ladite seconde portion d'aiguille (2) étant inclinée par rapport audit axe (18) de l'éprouvette (4), de manière que lors de l'usage l'écoulement de sang en sortie frappe seulement la paroi latérale (4a) de l'éprouvette.

2. Aiguille selon la revendication 1, caractérisée en ce que ledit tronçon intermédiaire (12) est situé dans un élément de protection (15) qui se trouve entre ledit élément d'emboîtement et retenue (5) pourvu d'un bord annulaire (14) destiné à assurer l'étanchéité dudit élément de couverture (6),et un corps annulaire (10) pour l'emboîtement et la retenu dudit respectif élément de couverture (11) lequel agit en tant qu'arrêt et retient le corps fileté (7) en engagement étanche avec ledit cylindre de support d'éprouvette (9).

3. Aiguille selon la revendication 3, caractérisée en ce que ledit corps annulaire (10) est de forme tronconique.

4. Aiguille selon la revendication 1, caractérisée en ce que ladite seconde portion d'aiguille (2) est inclinée vers le bas, en direction de l'axe de la première portion d'aiguille (1) et présente une coupure (2a) orientée vers le bas et s'étentdant le long d'un plan perpendiculaire au plan passant par les axes desdites portions d'aiguilles (1) et (2).
